# EUROPEAN PATENT APPLICATION

(11) **EP 4 722 674 A1**
(43) Date of publication of application: **08.04.2026**
(21) Application number: 25199277.2
(22) Date of filing: 01.09.2025
(51) Int. Cl.: G01L 19/00, G01L 19/06

(54) **PROTECTIVE ASSEMBLY FOR SENSORS**

(30) Priority: 04.10.2024 IN 202411075196
(71) Applicant: HONEYWELL INTERNATIONAL INC., Charlotte, NC 28202 (US)
(72) Inventor: H B, Akshay, Charlotte, 28202 (US); JAIN, Rajani Niranjan, Charlotte, 28202 (US); HEGDE, Santosh Nagapati, Charlotte, 28202 (US); MAUT, Sandeep Laxman, Charlotte, 28202 (US)
(74) Representative: Haseltine Lake Kempner LLP

(57) **Abstract**

Apparatuses, systems, and methods are provided for protective assemblies for sensors. **In** some embodiments, a protective assembly comprises a substrate. **In** some embodiments, the protective assembly further comprises at least one enclosing port adhered to the substrate. **In** some embodiments, the protective assembly further comprises one or more electronic components disposed within a cavity of the enclosing port and wire-bonded to the substrate. **In** some embodiments, the protective assembly further comprises a gel material disposed within the cavity such that it covers the one or more electronic components. **In** some embodiments, the protective assembly further comprises a fluoropolymer polyimide film (FEP) disposed proximate to a surface of the gel material.

## Description

### FIELD OF THE INVENTION

Embodiments of the present disclosure generally relate to protective assemblies comprising films, such as fluoropolymer polyimide films (FEPs), for sensors.

### BACKGROUND

Some types of medical devices, such as continuous positive airway pressure (CPAP) devices, rely on sensors (e.g., pressure sensors) to operate. Assemblies comprising such sensors may be exposed to contaminants (e.g., bodily fluids). In some examples, cleaning such assemblies may damage them and/or the sensors they comprise. In some examples, hard-gel-soft-gel packages may be used for protecting the sensors. However, such protective packages may affect accuracy of the sensors and/or calibration of the sensors. Applicant has identified many technical challenges and difficulties associated with such protective packages and/or pressure sensor assemblies. Through applied effort, ingenuity, and innovation, many of these identified problems have been solved by developing solutions that are included in embodiments of the present disclosure, many examples of which are described in detail herein.

### BRIEF SUMMARY

Various example embodiments described herein relate to protective assemblies comprising films (e.g., thin films), such as fluoropolymer polyimide films (FEPs), for sensors.

In accordance with various embodiments of the present disclosure, an apparatus is provided. In some embodiments, the apparatus comprises: (i) a substrate; (ii) at least one enclosing port adhered to the substrate; (iii) one or more electronic components disposed within a cavity of the enclosing port and wire-bonded to the substrate; (iv) a gel material disposed within the cavity such that it covers the one or more electronic components; and/or (v) a fluoropolymer polyimide film (FEP) disposed proximate to a surface of the gel material.

In some embodiments, the gel material is configured to protect the one or more electronic components and their electrical connections.

In some embodiments, the surface of the gel material on which the FEP is disposed is opposite one or more surfaces of the gel material proximate to the one or more electronic components.

In some embodiments, the one or more electronic components comprise at least one of a sensing element or an application-specific integrated circuit (ASIC).

In some embodiments, the gel material and the FEP form a biocompatible stacked gel column configured to impart a received pressure to the sensing element.

In some embodiments, the stacked gel column is substantially incompressible such that it is configured to impart substantially a same received pressure to the sensing element.

In some embodiments, the ASIC is configured to output a pressure sensed by the sensing element to a device.

In accordance with various embodiments of the present disclosure, a system is provided. In some embodiments, the system comprises: (1) a fluid flow tube; and (2) a pressure sensor assembly comprising: (i) a substrate; (ii) at least one enclosing port adhered to the substrate; (iii) one or more electronic components disposed within a cavity of the enclosing port and wire-bonded to the substrate; (iv) a gel material disposed within the cavity such that it covers the one or more electronic components; and (v) a fluoropolymer polyimide film (FEP) disposed proximate to a surface of the gel material.

In some embodiments, the gel material is configured to protect the one or more electronic components and their electrical connections.

In some embodiments, the surface of the gel material on which the FEP is disposed is opposite one or more surfaces of the gel material proximate to the one or more electronic components.

In some embodiments, the one or more electronic components comprise at least one of a sensing element or an application-specific integrated circuit (ASIC).

In some embodiments, the gel material and the FEP form a biocompatible stacked gel column configured to impart a received pressure of a medium being transported by the fluid flow tube to the sensing element.

In some embodiments, the stacked gel column is substantially incompressible such that it is configured to impart substantially a same received pressure of the medium to the sensing element.

In some embodiments, the ASIC is configured to output a pressure sensed by the sensing element to a device.

In accordance with various embodiments of the present disclosure, a method is provided. In some embodiments, the method comprises: (i) printing a first adhesive at one or more locations on a substrate; (ii) coupling one or more electronic components to the substrate via the first adhesive; (iii) dispensing a second adhesive surrounding a region comprising the one or more electronic components; (iv) coupling at least one enclosing port to the substrate via the second adhesive; (v) dispensing a gel material within a cavity defined by the at least one enclosing port such that the gel material covers at least the one or more electronic components; and (vi) disposing a fluoropolymer polyimide film (FEP) proximate to a surface of the gel material.

In some embodiments, the surface of the gel material on which the FEP is disposed is opposite one or more surfaces of the gel material proximate to the one or more electronic components.

In some embodiments, the one or more electronic components comprise at least one of a sensing element or an application-specific integrated circuit (ASIC).

In some embodiments, the gel material and the FEP form a biocompatible stacked gel column configured to impart a received pressure of a medium being transported by the fluid flow tube to the sensing element.

In some embodiments, the stacked gel column is substantially incompressible such that it is configured to impart substantially a same received pressure of the medium to the sensing element.

In some embodiments, the ASIC is configured to output a pressure sensed by the sensing element to a device.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 is a cross-sectional view of an example protective assembly;
FIGS. 2A-2C are cross-sectional views of various components of the example protective assembly of FIG. 1;
FIG. 3 is a series of top-down views showing fabrication of the example protective assembly of FIG. 1; and
FIG. 4 is a flowchart of an exemplary method of fabricating an example protective assembly, in accordance with some embodiments of the present disclosure.

### DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," "bottom," "left," "right," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "example" or "exemplary" is not necessarily to be construed as preferred or advantageous over other implementations.

The term "electrically coupled," "electrically coupling," "electrically couple," "electrically connected," "electrically connecting," "electrically connect," "in communication with," or "in electronic communication with" in the present disclosure refers to two or more elements or components being connected through wired means and/or wireless means, such that signals, electrical voltage/current, data and/or information may be transmitted to and/or received from these elements or components.

The term "in fluid communication with" in the present disclosure refers to two or more elements or components being connected through one or more paths or pathways, such that a fluid or other flowing media may be input to and/or output from these elements or components.

The term "component" may refer to an article, a device, or an apparatus that may comprise one or more surfaces, portions, layers and/or elements. For example, an example component may comprise one or more substrates that may provide underlying layer(s) for the component and may comprise one or more elements that may form part of and/or are disposed on top of the substrate. In the present disclosure, the term "element" may refer to an article, a device, or an apparatus that may provide one or more functionalities.

The term "sensor" refers to a component that may detect, measure, and/or identify any one or more attributes or characteristics of an environment of media, including but not limited to pressure(s).

In some examples, devices disclosed and/or discussed herein may comprise sensors (e.g., such as pressure sensors) and may further comprise protective assemblies for such sensors. An example of such a device is a continuous positive airway pressure (CPAP) device, which may be configured to aid a user with breathing, for example, during sleep. The CPAP device may comprise a fluid flow tube configured to transport air to the user via a mask, for example. To maintain accuracy and safety of medical devices such as CPAP devices, cleaning of various components of the medical devices (e.g., a fluid flow tube of a CPAP device) may be relied upon. However, in some cases, cleaning may be abrasive and/or damaging to components of the medical devices (e.g., sensors, protective assemblies for sensors, etc.).

Embodiments of the present disclosure, in some examples, provide apparatuses, systems, and methods for protective assemblies, for sensors, comprising films, for example, such as thin films. In some examples, a protective assembly may include a gel material and a thin film disposed on top of the gel material (wherein "on top of" indicates that the thin film is disposed proximate to an exposed surface of the gel material). In some examples, the thin film is a fluoropolymer polyimide film (FEP) and/or other types of film.

Example embodiments of the present disclosure, in some examples, may include a substrate. The substrate may be a printed circuit board (PCB) and/or may be comprised of other materials. In some examples, example embodiments of the present disclosure may include at least one enclosing port adhered to the substrate. The at least one enclosing port may be a substantially cylindrical shell. For example, the enclosing port may be configured to enclose an amount of gel or other compressible or incompressible (e.g., substantially incompressible) material.

In some embodiments, the protective assembly further comprises one or more electronic components disposed within a cavity of the enclosing port and wire-bonded to the substrate. In some embodiments, the protective assembly further comprises a gel material dispensed such that it covers the one or more electronic components. In some embodiments, the protective assembly further comprises a fluoropolymer polyimide film (FEP) disposed proximate to a surface of the gel material.

As described herein, embodiments of the present disclosure, in some examples, provide apparatuses, systems, and/or methods for protective assemblies, such as protective assemblies comprising thin film(s).

To address challenges and limitations associated with protective assemblies for sensors, various examples of the present disclosure may be provided. For example, various examples of the present disclosure may provide example apparatuses, systems, and/or methods for a protective assembly including a protective stack comprising an FEP film and a gel material.

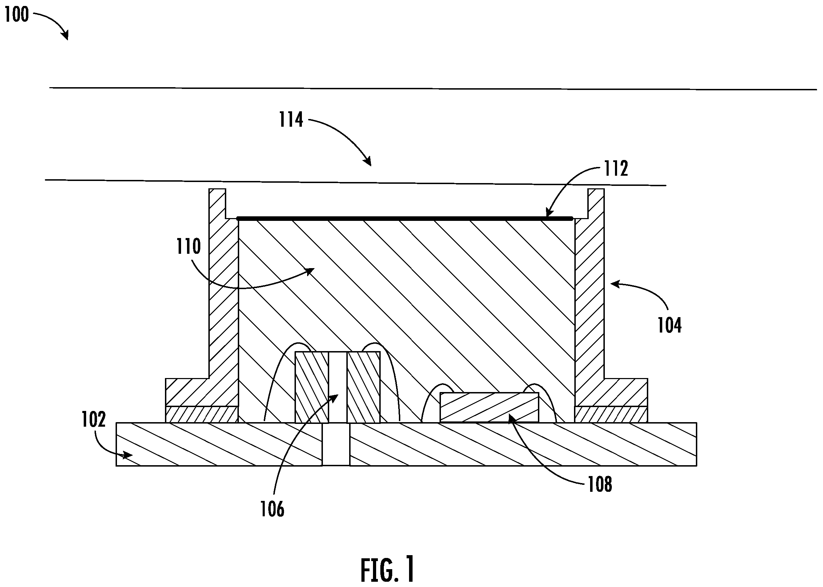

Referring now to FIG. 1, a cross-sectional view of an example protective assembly 100 of a medical device is provided. The assembly 100 comprises a substrate 102, an enclosing port 104, a sensing element 106, an application-specific integrated circuit (ASIC) 108, a gel material 110, and a thin film 112. The medical device comprising the assembly further comprises a fluid flow tube 114. Although the example of FIG. 1 shows one substrate, one enclosing port, one sensing element, one ASIC, one gel material, one thin film, and one fluid flow tube, any number of such components may present in a device and/or protective assembly.

The substrate 102 may be comprised of various materials. For example, the substrate 102 may be a printed circuit board (PCB) and/or other materials. The substrate 102 may be a PCB panel configured to comprise one or more circuits. For example, the assembly 100 may be an implementation of one such circuit comprised by a PCB panel.

The enclosing port 104 may be a substantially cylindrical shell. For example, the enclosing port 104 may be a substantially cylindrical element defining a cavity at approximately its center. The enclosing port 104 may further comprise one or more flared features for increasing surface area to be adhered to the substrate 102. In some examples, the enclosing port 104 may be coupled to the substrate 102 via one or more adhesives. For example, the one or more adhesives may be dispensed surrounding a predetermined area (e.g., an area comprising one or more electrical components such as the sensing element 106, the ASIC 108, and/or other components).

The sensing element 106 may be a sensing element configured to measure pressure, force, and/or other properties of a medium. For example, the sensing element 106 may be a pressure sensor. The sensing element 106 may be wire-bonded to the substrate 102.

The ASIC 108 may be configured to perform predetermined actions and/or processes. For example, the ASIC 108 may be configured to receive information (e.g., from the sensing element 106), transmit information (e.g., to another device) and/or the like. The ASIC 108 may be wire-bonded to the substrate 102.

The gel material 110 may be disposed within the cavity defined by the enclosing port 104 such that the gel material 110 covers and/or surrounds the one or more electrical components of the assembly 100 (e.g., the sensing element 106, the ASIC 108, and/or other components). In some examples, the gel material 110 extends at least a height past the height of the sensing element 106, the ASIC 108, and/or electrical connections (e.g., the respective wire-bonds). Advantages of the gel material 110 may include, in some examples, protecting the one or more electronic components and/or their electrical connections. The gel material 110 may be further configured to impart a received pressure and/or force to the sensing element 106.

The thin film 112 may be disposed proximate to an exposed surface of the gel material 110. The thin film 112 may be comprised of various materials. In some embodiments, the thin film 112 is a fluoropolymer polyimide film (FEP). The thin film 112 may be described as a "thin" film based on having a thickness of approximately 0.5 mm or less (e.g., such as 0.0127 mm for FEP). Advantages of an FEP thin film 112 include, in some examples: improved electrical insulation and dielectric strength (e.g., approximately 4400 V/mm or more) compared with values for other types of films (e.g., dielectric strength of approximately 2200 V/mm); being heat bondable and sealable; having high bond strength ranging from approximately 1.0 N/mm² to approximately 10 N/mm² (e.g., preferably from approximately 3.0 N/mm² to 6.5 N/mm²); being usable at high and/or low temperatures ranging from approximately 0 degrees Celsius to approximately 50 degrees Celsius, preferably from approximately -20 degrees Celsius to approximately 125 degrees Celsius, more preferably from approximately -269 degrees Celsius to approximately 400 degrees Celsius; protecting against radiation; protecting against damage and/or contamination; and/or other advantages. In some examples, the thin film 112 may be further configured to protect the gel material 110 (and/or the electrical components protected by the gel material 110) from abrasions such as those arising from cleaning brushes, for example.

The fluid flow tube 114 may be configured to transport a medium, for example, such as the medium being measured by the sensing element 106. In some embodiments, the fluid flow tube 114 is configured to transport air from a CPAP device to a user. The fluid flow tube may be disposed proximate to the assembly 100. For example, the enclosing port 104 may protrude a height past the thin film 112 such that the fluid flow tube 114, when positioned proximate to the assembly 100, is spaced at least a distance away from the thin film 112.

In some examples, the medium being transported by the fluid flow tube 114 may expand the diameter of the fluid flow tube 114 such that an outer surface of the fluid flow tube 114 comes into contact with the thin film 112. Based on the fluid flow tube 114 imparting a pressure onto the thin film 112, the thin film 112 may then impart the same pressure to the gel material 110. The gel material 110 may then impart the same pressure to the sensing element 106. The sensing element 106 may transmit information indicating the measured pressure, for example, to the ASIC 108 and/or to other components coupled to the substrate 102.

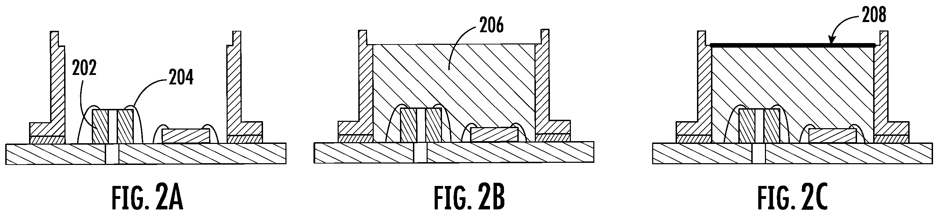

FIGS. 2A-2C show cross-sectional views of various components of the example protective assembly of FIG. 1, for example, as part of a fabrication process.

Referring now to FIG. 2A, a cross-sectional view of a partially fabricated protective assembly is provided. A substrate coupled, via adhesive, to an enclosing port is shown. An electronic component 202 (e.g., a sensing element such as the sensing element 106, an ASIC such as the ASIC 108, and/or the like) may be coupled to the substrate, for example, via adhesive. Electrical connections 204 (e.g., wire bonds) may electrically couple the electronic component 202 to the substrate.

Referring now to FIG. 2B, a cross-sectional view of a partially fabricated protective assembly is provided. A substrate coupled to an enclosing port, surrounding electronic component(s) electrically coupled to the substrate, is shown. A gel material 206 may be dispensed within a cavity defined by the enclosing port such that the gel material 206 covers and extends past the electronic component(s) 202 and the electrical connections 204, without extending past the height of the enclosing port.

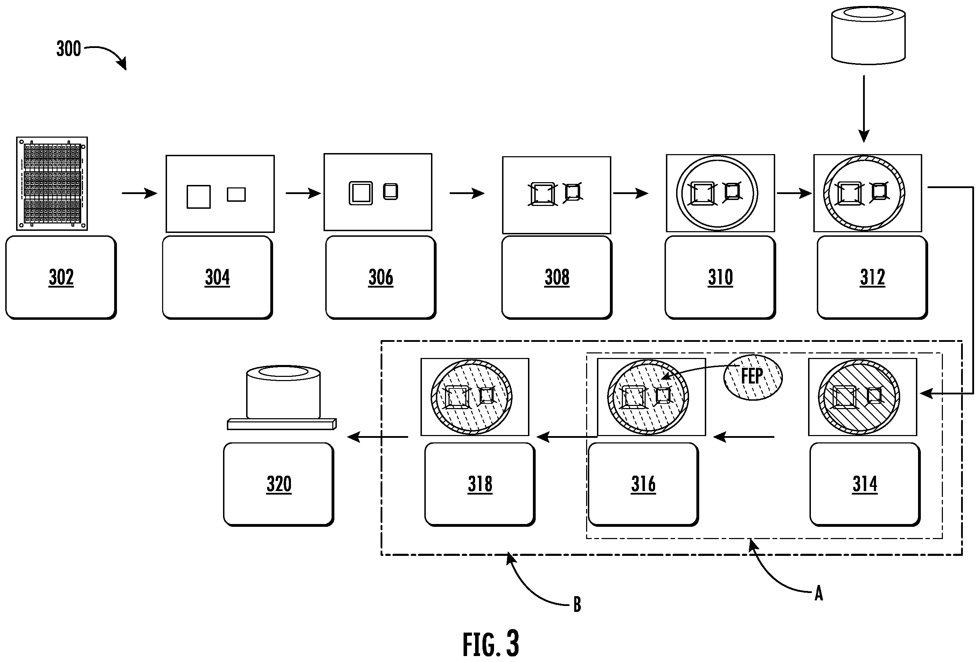

Referring now to FIG. 3C, a cross-sectional view of a protective assembly is provided. A substrate coupled to an enclosing port, comprising a gel material covering electronic component(s) electrically coupled to the substrate, is shown. A thin film 208, such as an FEP, may be disposed proximate to the previously exposed surface of the gel material 206. The thin film 208 may be bonded (e.g., heat bonded) to the gel material 206. The protective assembly may be cured such that the gel material 206 becomes cured and/or such that a bond between the thin film 208 and the gel material 206 is formed.

Referring now to FIG. 3, a series 300 of top-down views showing fabrication of the example protective assembly of FIG. 1 is provided.

At step/operation 302, a PCB panel may be provided. In some examples, the PCB panel may comprise a plurality of sensors (wherein "sensor" refers to a package comprising a protective assembly as described herein). The PCB panel may comprise any number of sensors.

Steps/operations 304-320 depict fabrication of a single sensor of the PCB panel.

At step/operation 304, adhesive(s) may be dispensed, for example, via printing onto the PCB panel. The adhesive(s) may be dispensed at one or more locations, for example, to accommodate a corresponding quantity of electronic components.

At step/operation 306, one or more electronic components may be disposed proximate to their corresponding adhesive(s). The one or more electronic components may be placed via pick-and-place and/or other methods. For example, a sensing element and an ASIC may be placed on the printed adhesive(s) on the PCB panel. The existing assembly may then undergo oven curing, argon plasma cleaning, and/or other processes.

At step/operation 308, one or more electrical connections between the one or more electronic components and the PCB panel may be performed. For example, the one or more electronic components may be wire-bonded to the PCB panel.

At step/operation 310, adhesive(s) may be dispensed (e.g., in a substantially circular geometry) onto the PCB panel surrounding the electronic components.

At step/operation 312, an enclosing port may be coupled to the PCB panel via the adhesive dispensed at step/operation 310. The existing assembly may then undergo isopropyl alcohol (IPA) cleaning, baking, oxygen plasma cleaning, and/or other processes.

Sub-process B comprises steps/operations 314, 316, and 318, wherein sub-process A comprises steps/operations 314 and 316.

Referring now to sub-process A, a single piece process flow is provided. At the start of sub-process A, vacuum degassing, oven curing, and/or other processes may be performed. Sub-process A may be performed in a vacuum chamber. At step/operation 314, a gel material may be dispensed within the enclosing port such that the gel material covers the electronic components and the electrical connections. In some examples, the gel material may be dispensed by an X-Y dispenser. At step/operation 316, an FEP thin film may be coupled to the gel material. The FEP thin film may be placed proximate to the exposed surface of the gel material via pick-and-place and/or other methods. The existing assembly may then undergo oven curing.

Referring now to the remaining portion of sub-process B, step/operation 318 is shown. At step/operation 318, the sensor may be calibrated. The FEP thin film may be configured to protect the gel material, the electronic components, and/or the electrical connections during calibration.

At step/operation 320, singulation and/or packaging of the sensor may be performed.

Steps 304-320 may be performed simultaneously and/or successively for a plurality of sensors of the PCB panel.

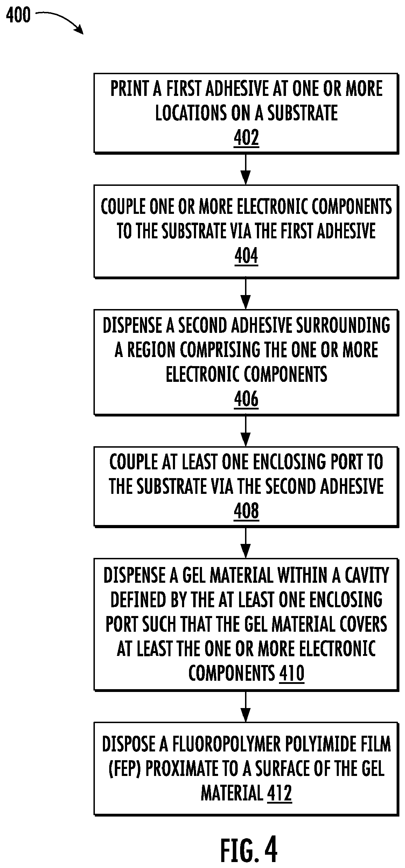

Referring now to FIG. 4, a flowchart of an exemplary method 400 of fabricating an example protective assembly is provided.

At step/operation 402, a first adhesive may be printed at one or more locations on a substrate.

At step/operation 404, one or more electronic components may be coupled to the substrate via the first adhesive.

At step/operation 406, a second adhesive may be dispensed surrounding a region comprising the one or more electronic components.

At step/operation 408, at least one enclosing port may be coupled to the substrate via the second adhesive.

At step/operation 410, a gel material may be dispensed within a cavity defined by the at least one enclosing port such that the gel material covers at least the one or more electronic components.

At step/operation 412, a fluoropolymer polyimide film (FEP) may be disposed proximate to an exposed surface of the gel material.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

While various embodiments in accordance with the principles disclosed herein have been shown and described /above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some cases one or more of the steps depicted may occur substantially simultaneously, or additional steps may be involved. Although specific terms are employed herein, they are used in a generic and descriptive sense only and not for purposes of limitation.

## Claims

1. An apparatus comprising:
a substrate;
at least one enclosing port adhered to the substrate;
one or more electronic components disposed within a cavity of the enclosing port and wire-bonded to the substrate;
a gel material disposed within the cavity such that it covers the one or more electronic components; and
a fluoropolymer polyimide film (FEP) disposed proximate to a surface of the gel material.

2. The apparatus of claim 1, wherein the gel material is configured to protect the one or more electronic components and their electrical connections.

3. The apparatus of any of claims 1 to 2, wherein the surface of the gel material on which the FEP is disposed is opposite one or more surfaces of the gel material proximate to the one or more electronic components.

4. The apparatus of any of claims 1 to 3, wherein the one or more electronic components comprise at least one of a sensing element or an application-specific integrated circuit (ASIC).

5. The apparatus of any of claims 1 to 4, wherein the gel material and the FEP form a biocompatible stacked gel column configured to impart a received pressure to the sensing element.

6. The apparatus of any of claims 1 to 5, wherein the stacked gel column is substantially incompressible such that it is configured to impart substantially a same received pressure to the sensing element.

7. The apparatus of any of claims 1 to 6, wherein the ASIC is configured to output a pressure sensed by the sensing element to a device.

8. A system comprising:
a fluid flow tube; and
a pressure sensor assembly comprising:
a substrate;
at least one enclosing port adhered to the substrate;
one or more electronic components disposed within a cavity of the enclosing port and wire-bonded to the substrate;
a gel material disposed within the cavity such that it covers the one or more electronic components; and
a fluoropolymer polyimide film (FEP) disposed proximate to a surface of the gel material.

9. The system of claim 8, wherein the gel material is configured to protect the one or more electronic components and their electrical connections.

10. The system of any of claims 8 to 9, wherein the surface of the gel material on which the FEP is disposed is opposite one or more surfaces of the gel material proximate to the one or more electronic components.

11. The system of any of claims 8 to 10, wherein the one or more electronic components comprise at least one of a sensing element or an application-specific integrated circuit (ASIC).

12. The system of any of claims 8 to 11, wherein the gel material and the FEP form a biocompatible stacked gel column configured to impart a received pressure of a medium being transported by the fluid flow tube to the sensing element.

13. The system of any of claims 8 to 12, wherein the stacked gel column is substantially incompressible such that it is configured to impart substantially a same received pressure of the medium to the sensing element.

14. The system of any of claims 8 to 13, wherein the ASIC is configured to output a pressure sensed by the sensing element to a device.

15. A method comprising:
printing a first adhesive at one or more locations on a substrate;
coupling one or more electronic components to the substrate via the first adhesive;
dispensing a second adhesive surrounding a region comprising the one or more electronic components, wherein the one or more electronic components comprise at least one of a sensing element or an application-specific integrated circuit (ASIC);
coupling at least one enclosing port to the substrate via the second adhesive;
dispensing a gel material within a cavity defined by the at least one enclosing port such that the gel material covers at least the one or more electronic components; and
disposing a fluoropolymer polyimide film (FEP) proximate to a surface of the gel material;
wherein the surface of the gel material on which the FEP is disposed is opposite one or more surfaces of the gel material proximate to the one or more electronic components; and
wherein the gel material and the FEP form a substantially incompressible biocompatible stacked gel column configured to impart a received pressure of a medium being transported by the fluid flow tube to the sensing element.
